# EUROPEAN PATENT SPECIFICATION

(11) **EP 0 735 845 B1**
(45) Date of publication and mention of the grant of the patent: **31.07.2002**
(21) Application number: 95904734.1
(22) Date of filing: 22.12.1994
(51) Int. Cl.: A61F 2/24, A61B 17/00, A61B 19/02

(54) **CARDIAC VALVE HOLDERS**
HERZKLAPPENHALTER
SUPPORTS DE VALVULES CARDIAQUES

(30) Priority: 22.12.1993 SE 9304234; 13.09.1994 SE 9403043
(43) Date of publication of application: 09.10.1996
(73) Proprietor: ST. JUDE MEDICAL INC., St. Paul, 55117 Minnesota (US)
(72) Inventor: Bugge, Mogens, 413 08 Göteborg (SE)
(74) Representative: Kenyon, Sarah Elizabeth
(86) International application number: PCT/SE94/01247
(87) International publication number: WO 95/17139

(56) References cited:
- WO-A-92/12688
- GB-A- 2 181 057
- US-A- 3 409 013
- US-A- 3 828 787
- US-A- 5 201 880
- US-A- 5 236 450

## Description

The present invention refers to a holder for artificial heart valves, of the type which is firmly but detachably attached to the valve and which is intended to be removed first after that the valve is correctly placed or operated inside, by breaking a transportation safety member whereby the partially foldable holder can be removed.

### BACKGROUND OF THE INVENTION

Within cardiac surgery the implantation of artificial heart valves cover about 20% of operations. Considering the entire world, this implies implantation of at least 100000 valves per year.

Heart valves are supplied in different sizes from factories in small transportation containers. For each product there is a prosthesis tester to measure which size to fit into the actual heart. When the test is finished, the actual size and type of valves are produced and the implantation is carried out.

The holder for artificial heart valves is used for mechanical valves and for biological valves. The purpose of the holder is to:
1) fix the valve in its container during the transportation, and
2) serve as a handle for a cardiac surgeon and his operation team during the implantation.

Such holders are known from e.g. WO-A-92/12688 and US-A-3 828 787.

There are two main types of valves: a) aortic valves and b) mitral valves. Moreover, these are produced in several different sizes.

If the wrong size or type of valve is used, results can be disastrous, therefore the identification of the valves is surrounded by security procedures at the operation time.

It is consequently very important that the valves, which are much alike, are implanted in the heart in the correct way and in the correct position, since an incorrectly placed valve can cause the death of the patient. Therefore aortic valves and mitral valves should not be allowed to be mixed, which can happen in several ways. A method is to implant a mitral valve in aortic position, or the aortic valve in mitral position. Another method is to implant a valve turned.wrong way (up and down), which is possible in both positions.

Another type of error that can occur is wrong packing of the product at the factory. Usually, there is an adhesive label on the transport can, which indicates the content. At the factory the packaging is surrounded by several security routines to ensure that the content of the container corresponds to the text, which the adhesive label on the container indicates. Despite this, wrong packaging can occur as well. For example, a valve of size 25 can be put in a container with size indication 27 and an operative mistake can be the consequence, if the mistake is not detected through control in the operation theatre.

Another problem with operation of the heart valve is that the holder or the prosthesis tester breaks down and fragments of these have been dropped and are left inside the heart. Also, it has occurred that the entire holder has been forgotten in the heart, in the belief that it is a part of the heart valve.

### THE OBJECTS OF THE INVENTION AND MOST IMPORTANT FEATURES

The object of the present invention is to provide a holder for the heart valve that as much as possible eliminates or reduces such mistakes as mentioned above. Another object of the invention is to prevent the wrong packaging of the valves provided with the holder already at the production stage, and a third object is to be able to detect a holder or a part thereof, which during the operation could have been dropped. These tasks have been solved through the features stated in the claims.

Thus, according to the present invention, there is provided a holder for an artificial heart valve, said holder comprising a holder body for coupling to a heart valve and characterised by further comprising a safety member adapted to correspond with a profile of a heart valve and to prevent said coupling if said safety member does not correspond with said profile.

### DESCRIPTION OF DRAWINGS

The invention will be described closer below using some embodiments, with reference to attached drawings.
Fig. 1 schematically shows, in a supernatural size, a cut through a conventional standard holder mounted in an artificial aortic valve.
Fig. 2 shows a cut, analogous to Fig. 1, through a holder with a heart valve for aortic position according to the present invention.
Fig. 3 shows a cut through a conventional standard holder for a mitral valve.
Fig.4 shows a cut, analogous to fig. 3, through a holder with a heart valve for mitral position according to the present invention placed in a transportation container.
Fig. 5 shows a cut through a holder with heart valve according to a modified embodiment, which is provided with a safety ring and placed in a transportation container.
Fig. 6 shows a holder according to figure 2 with an aortic prosthesis rigidly sewn to the heart valve.
Fig. 7 shows a holder for a biological heart valve in the aortic position.

### DESCRIPTION OF THE EMBODIMENTS

The invention is exemplified below for mechanical valves of double blade type, but the same principles can of course be applied to any type of valve holders.

The heart valve itself consists of a valve ring 10, in which two semicircular valve blades 11 are mounted, each on a joint axis 12. Exterior to the valve ring 10 there is a valve cuff 13, which is the 'soft' part of the valve, which is sewn firmly (sutured) on the heart.

A valve holder 9 in this example consists of two parts 14 and 15, which are mutually united through a joint 16. The holder 9 is mounted on the valve ring 10 with the valve blade in an open position and after mounting the two parts are held together by a suture 17 around the two handle portions 18,19 of the holder. To be able to remove the holder from the valve, suture 17 is cut and the holder is opened inwardly through the joint 16.

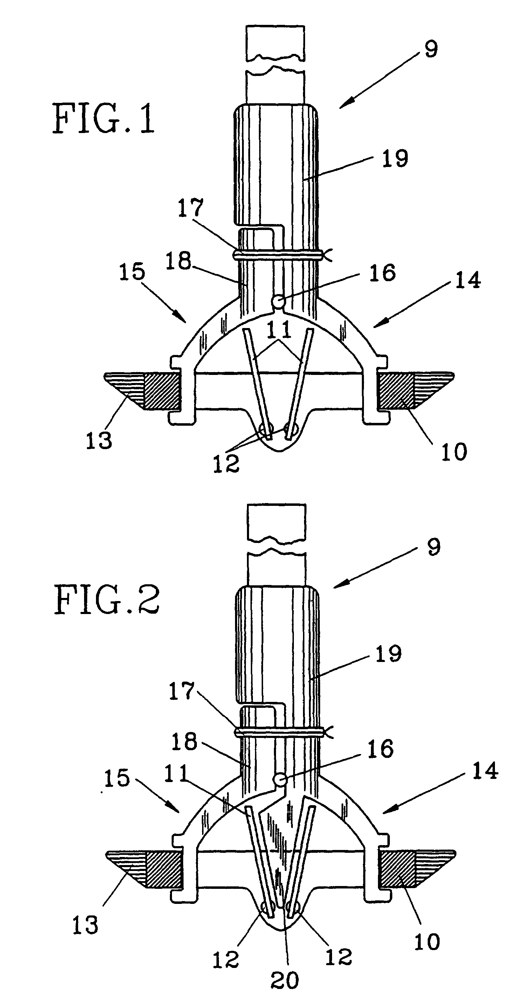

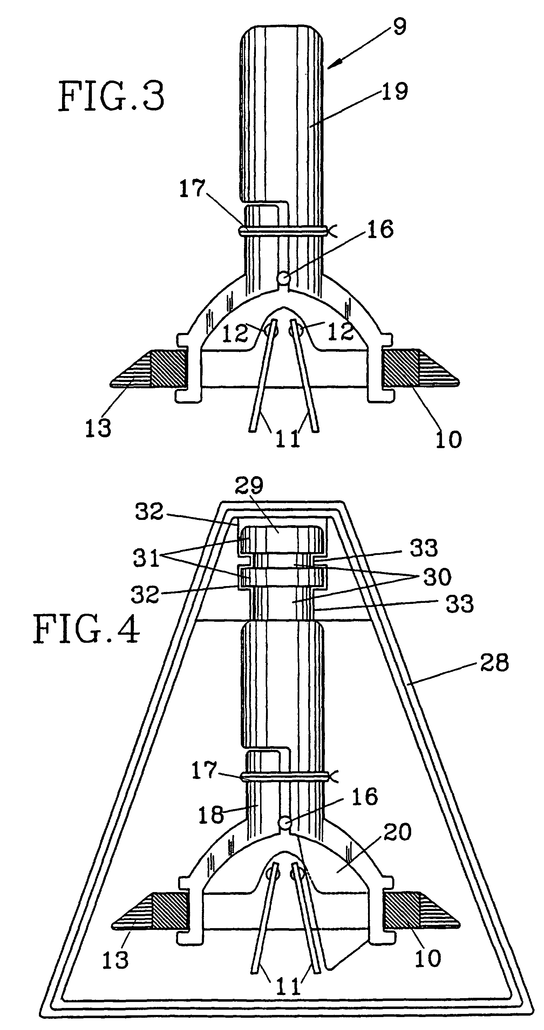

As it appears from fig. 2 safety means 20 is provided by means of an extension of the handle portion 19, which continues downwards between two blades 11 of the heart valve in the aortic case, (Fig. 2), and along one side of a blade 11 in the mitral case (Fig. 4) respectively.

Preferably, the holder 9 for the aortic and mitral valves, is provided with different colours or colour codes, e.g. red for aortic holder and blue for a mitral holder. This will be a very clear indication for a person who handles the valve, who is informed about the holder in question. If the person in question is colour-blind or does not know the colour coding, the holder and the valve are marked with identification symbols on the side of the packaging. The holder 9 is designed such that it only fits into in the valve in one way.

The holders for heart valves are relatively simple objects, generally manufactured of plastics. The holder is manufactured in one or two parts and concerning the mechanical valve, the holder fits exactly in the opening of the valve, while the holders for biological valves are sutured onto one or other side of the valve.

Conventional holders for the double bladed heart valves face the problem that they can be mounted from both sides of the valve. When one handles an aortic valve, the holder must be placed on the opening side of the valve, while a mitral valve holder must be placed on the valve closing side. If the valve is placed the wrong way up in the heart, the passage is closed and the heart cannot pump. If the error is detected by the surgeon, the valve must be removed and placed in the correct position, otherwise the patient cannot survive the operation.

To prevent an aortic valve holder being mounted from the wrong side the safety means 20, according to the present invention, is designed such that it only fits from the opening side. Although such holders are only manufactured in one colour, mistakes can be eliminated to a considerable extent. To prevent a mitral valve holder being mounted from the wrong side in a similar method the safety means 20 is designed on the mitral holder so that the holder can only be mounted from the closing side.

Although the labels of different types are put on the packaging or on the valve itself, they are no guarantees that correct valve size is used. According to the invention, the size of the valve ring is decided by the holder, which besides having a visible number infused in the plastic at the production, it also has the same colour marking as the prosthesis tester.

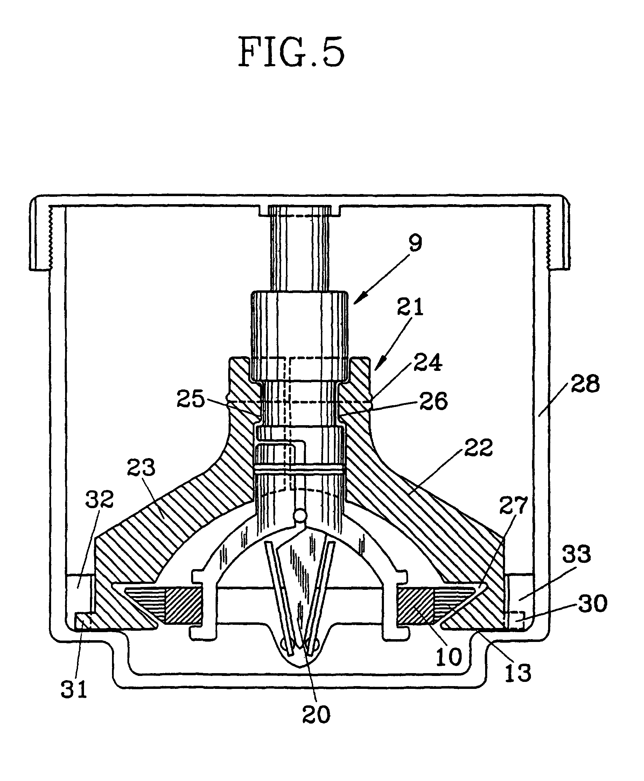

To further ensure that a wrong size indication on the valve is not mounted on the holder, a safety ring 21 is placed over the valve cuff 13 of the heart valve, as it is suggested in fig. 5. The safety ring 21 is made of two halves 22, 23 which are held together by a suture 24 and are removed before the valve is sutured firmly on the heart, while the holder 9 itself is removed after the valve has been placed in its end position in the heart.

The safety ring 21 is designed to fit only to one size of the holder. For example, by means of varying length L of a peripheral recess 25 and a corresponding projecting part 26 in the safety ring 21, where the length "L" is different for different valve sizes, the confusions can be avoided. If the holder is made in such a way, a larger safety ring 21 cannot fit in the recess 25. Mounting a smaller ring in the holder is also not possible, since the outer dimension of the valve is larger than that of the safety ring, which consequently will not fit across the valve cuff 13. Furthermore, on the holder as well as on the safety ring a dimension number is infused in plastic and they are of same colour.

As it appears from Fig. 5, the safety ring 21 has an internal recess 27, the form of which fits the valve cuff 13. Since cuffs in mitral and aortic valves are different, usually with a larger cuff in the mitral valves and with different inclination profiles on the side, which is turned against the heart, the safety ring will be a guarantee that right type of valve is mounted on the holder. Thus, with this system a mitral valve cannot be mounted in an aortic holder by mistake and an aortic valve cannot be mounted in a mitral holder, which is the case with present existent systems.

If the holder is manufactured in this way, this guarantees that only valves of the same size can be mounted on the holder, which is not the case with present existing systems. This is specially important, since the size of the valve, which is chosen by the surgeon, is usually critical and the size is not marked on the valve itself, but generally only on the packaging.

The invention is very simple and costs caused by the change of the holder and the outward ring are minimal compared with the problems that a simple mistake could cost (and mistakes have been occurring more than once). The new holders will not negatively effect the valves in any other way than that until now.

Generally, artificial heart valves are supplied in transportation container 28 with the holder mounted in the valve. The holder has a double function, in that during transportation it fixes the valve, and during the suture procedure it is usable in the heart as a practical little lever.

As it appears from fig. 4 the shaft 29 of the holder is equipped with one or more outer fitting elements, which in this embodiment consists of peripheral recesses 30 and bars 31, which have different width for every desired type and size of holder. The transportation container 28 is provided at production with corresponding recesses 32 and bars 33. By varying the size and the number of the recesses and the distance between these, endless combinations are obtained, which allow only one special type of valve holder to fit the container. Thanks to that, the valve holder cannot simply fit in the container without being the correct type, the confusion is thereby prevented already at the packaging in the factory before distribution of the possible wrongly identified product.

The recesses 30 and the bars 31 can also be used for identification of the holder in the production, if the tool handling the product is provided with corresponding details, in case someone so desires.

Also, the safety ring 21 can be provided with a fitting element, which is developed so that only the correct type of valve can fit into the container 28, which is exemplified in fig. 5.

By providing the valve holder with these simple fitting elements the risk of a valve being placed in a wrong container is eliminated, assuming that the valve holder from the beginning is constructed so that only a right valve can be mounted on the holder.

Due to different causes a holder or a part thereof may be left in the area of the operation and cause serious problems. To be able to discover that some strange object of mentioned type is left in the body after the operation, preferably, the holder is made of a material which becomes visible by x-ray on an x-ray plate or screen. Another possibility is to add micro particles, having enough density that it can be detected by means of x-ray, to the plastic material during the manufacturing the holder. A third possibility is that to the exterior of the holder apply (infuse) small plates or the like, which become visible by means of x-ray. It is also possible to "mark" the holder using radioactivity, so that it can be detected if it should be left in the body.

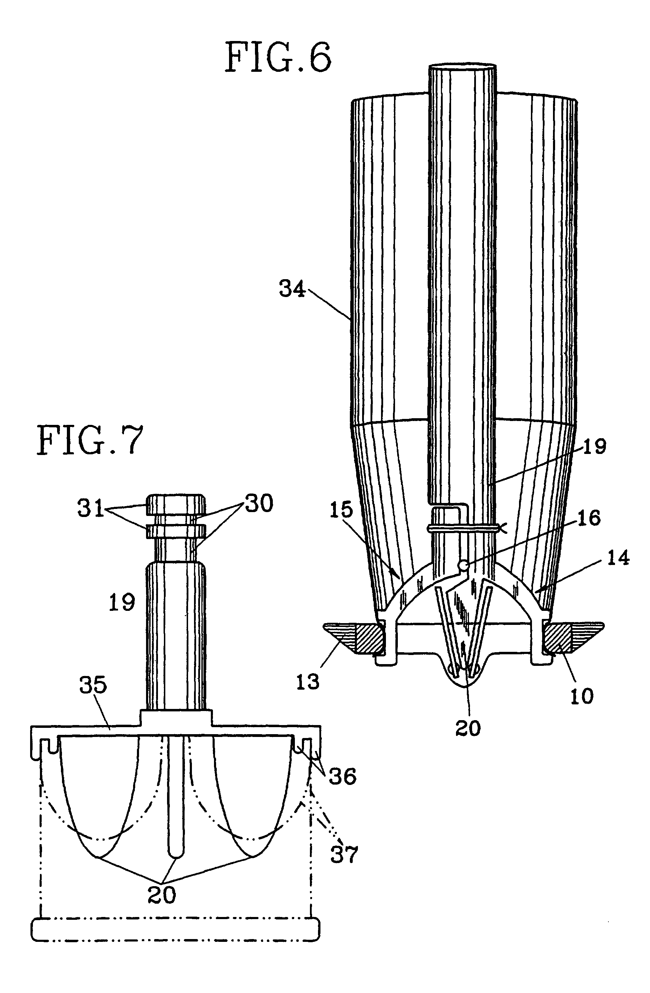

When changing the heart valve it can also be desired to change the part of the aorta that is directly connected to the valve. Fig. 6 shows a holder specially constructed for this purpose, which is provided with an extended handle portion 19 that is some how longer than the aortic prosthesis 34.

In certain cases biological heart valves are used, which require special holders, as shown in fig. 7, which comprises a base plate 35 with peripheral flanges 36 to which the biological valve 37 is rigidly sewn with some stitches. In same way, in other embodiments at the base plate 35 is provided safety means 20, which is provided to extend into the valve. At the handle portion 19, for instance there is provided fitting elements 30 and 31, for cooperation with corresponding fitting elements at the transportation container.

## Claims

1. A holder (9) for an artificial heart valve (10 - 13), said holder comprising a holder body (14, 15) for coupling to a heart valve (10 - 13) and **characterised by** further comprising a safety member (20) adapted to correspond with a profile of a heart valve and to prevent said coupling if said safety member does not correspond with said profile.

2. A holder according to claim 1, wherein said holder body (14, 15) comprises a first part and a second part assembled with each other by means of a joint (16) and wherein said first part comprises a handle.

3. A holder according to claim 2, wherein movement of said first and second parts relative to each other is prevented by means of a second safety member (17).

4. A holder according to claim 3, wherein said second safety member (17) is a suture.

5. A holder according to any one of claims 2 to 4, wherein said second part is fixed to said handle.

6. A holder according to any one of the preceding claims, further comprising a safety ring (21) adapted to be detachably mounted to said holder body (14, 15) and to surround the exterior of a heart valve, wherein the safety ring has a shape which corresponds to a shape of a periphery of a heart valve.

7. A holder according to claim 6, wherein said holder body comprises a recess and said safety ring comprises a projecting part adapted to fit in the recess.

8. A holder according to claim 6 or claim 7, wherein said holder is for use with a transportation container and wherein said holder comprises a first fitting element adapted to fit a container profile of a transportation container and to prevent said use of the holder with a transportation container if said first fitting element does not fit said container profile.

9. A holder according to claim 8, wherein said first fitting element comprises a bar adapted to fit a first recess of a transportation container.

10. A holder according to any one of the preceding claims, wherein the holder has a size and a shape and is marked by a colour corresponding to the size and shape.

11. A holder according to any one of the preceding claims, wherein the holder comprises material which is detectable through X-ray or through the emission of detectable radiation.

12. An operation set for cardiac surgery comprising an artificial or biological heart valve, a holder according to any one of the preceding claims, a prosthesis size tester and a transportation container wherein the heart valve, the holder and the prosthesis size tester are provided with the same colour and/or are marked by the same symbol.

## Patentansprüche

1. Halter (9) für eine künstliche Herzklappe (10 - 13), wobei der Halter einen Halterkörper (14, 15) zum Ankuppeln an eine Herzklappe (10 - 13) aufweist und **dadurch gekennzeichnet ist, dass** er ferner ein Sicherungselement (20) aufweist, das ausgelegt ist, um einem Profil einer Herzklappe zu entsprechen und um die Kupplung zu verhindern, wenn das Sicherungselement dem Profil nicht entspricht.

2. Halter nach Anspruch 1, worin der Halterkörper (14, 15) ein erstes Teil und ein zweites Teil aufweist, die miteinander mittels eines Gelenks (16) zusammengebaut sind, und worin das erste Teil einen Griff aufweist.

3. Halter nach Anspruch 2, worin eine Bewegung der ersten und zweiten Teile relativ zueinander mittels eines zweiten Sicherungselements (17) verhindert wird.

4. Halter nach Anspruch 3, worin das zweite Sicherungselement (17) ein Faden ist.

5. Halter nach einem der Ansprüche 2 bis 4, worin das zweite Teil an dem Griff befestigt ist.

6. Halter nach einem der vorhergehenden Ansprüche, der ferner einen Sicherungsring (21) aufweist, der ausgelegt ist, um an dem Halterkörper (14, 15) abnehmbar angebracht zu werden und um das Äußere der Herzklappe zu umgeben, worin der Sicherungsring eine Form hat, die einer Umfangsform einer Herzklappe entspricht.

7. Halter nach Anspruch 6, worin der Halterkörper eine Vertiefung aufweist und der Sicherungsring ein Vorsprungsteil aufweist, das dazu ausgelegt ist, in die Vertiefung zu passen.

8. Halter nach Anspruch 6 oder Anspruch 7, worin der Halter mit einem Transportbehälter verwendbar ist und worin der Halter ein erstes Passelement aufweist, das ausgelegt ist, um an ein Behälterprofil eines Transportbehälters zu passen und um die Verwendung des Halters mit einem Transportbehälter zu verhindern, wenn das erste Passelement nicht zu dem Behälterprofil passt.

9. Halter nach Anspruch 8, worin das erste Passelement eine Stange aufweist, die dazu ausgelegt ist, in eine erste Vertiefung eines Transportbehälters zu passen.

10. Halter nach einem der vorhergehenden Ansprüche, worin der Halter eine Größe und eine Form hat und durch eine der Größe und Form entsprechende Farbe markiert ist.

11. Halter nach einem der vorhergehenden Ansprüche, worin der Halter ein Material aufweist, das durch Röntgenstrahlen oder durch die Emission einer erfassbaren Strahlung erfassbar ist.

12. Operationsset für Herzoperationen, umfassend eine künstliche oder biologische Herzklappe, einen Halter nach einem der vorhergehenden Ansprüche, einen Prothesengrößentester und einen Transportbehälter, worin die Herzklappe, der Halter und der Prothesengrößentester mit der gleichen Farbe versehen sind und/oder durch das gleiche Symbol markiert sind.

## Revendications

1. Support (9) pour une valvule cardiaque artificielle (10 - 13), ledit support comprenant un corps de support (14, 15) pour être couplé à une valvule cardiaque (10 - 13), **caractérisé en ce qu'**il comprend en outre un élément de sécurité (20) adapté pour correspondre à un profil d'une valvule cardiaque et pour empêcher ledit couplage si ledit élément de sécurité ne correspond pas audit profil.

2. Support selon la revendication 1, dans lequel ledit corps de support (14, 15) comprend une première partie et une deuxième partie assemblées l'une avec l'autre au moyen d'un joint (16), et dans lequel ladite première partie comprend une poignée.

3. Support selon la revendication 2, dans lequel un déplacement desdites première et deuxième parties l'une par rapport à l'autre est empêché au moyen d'un deuxième élément de sécurité (17).

4. Support selon la revendication 3, dans lequel ledit deuxième élément de sécurité (17) est une suture.

5. Support selon l'une des revendications 2 à 4, dans lequel ladite deuxième partie est fixée à ladite poignée.

6. Support selon l'une des revendications précédentes, comprenant en outre une bague de sécurité (21) adaptée pour être montée de façon détachable sur ledit corps de support (14, 15) et pour entourer l'extérieur d'une valvule cardiaque, dans lequel la bague de sécurité présente une forme qui correspond à une forme d'une périphérie d'une valvule cardiaque.

7. Support selon la revendication 6, dans lequel ledit corps de support comprend un enfoncement et ladite bague de sécurité comprend une partie en saillie adaptée pour s'insérer dans l'enfoncement.

8. Support selon l'une des revendications 6 ou 7, dans lequel ledit support est destiné à l'utilisation avec un conteneur de transport et dans lequel ledit support comprend un premier élément de réception adapté pour recevoir un profil de conteneur d'un conteneur de transport et pour empêcher ladite utilisation du support avec un conteneur de transport si ledit premier élément de réception ne reçoit pas ledit profil de conteneur.

9. Support selon la revendication 8, dans lequel ledit premier élément de réception comprend une barre adaptée pour s'insérer dans un premier enfoncement d'un conteneur de transport.

10. Support selon l'une des revendications précédentes, dans lequel le support présente une taille et une forme et est marqué par une couleur correspondant à la taille et à la forme.

11. Support selon l'une des revendications précédentes, dans lequel le support comprend un matériau qui est détectable par rayons X ou par l'émission d'un rayonnement détectable.

12. Module d'opération pour chirurgie cardiaque comprenant une valvule cardiaque artificielle ou biologique, un support selon l'une des revendications précédentes, un testeur de la taille de la prothèse et un conteneur de transport dans lequel la valvule cardiaque, le support et le testeur de la taille de la prothèse sont réalisés avec la même couleur et/ou sont marqués avec le même symbole.
